(19) Europäisches Patentamt
European Patent Office
Office européen des brevets

(11) Veröffentlichungsnummer: **0 269 964 B1**

(12) **EUROPÄISCHE PATENTSCHRIFT**

(45) Veröffentlichungstag der Patentschrift: **29.01.92**

(51) Int. Cl.[5]: **C07C 45/50**, B01J 31/24

(21) Anmeldenummer: **87117134.4**

(22) Anmeldetag: **20.11.87**

(54) **Verfahren zur Herstellung von Aldehyden.**

(30) Priorität: **27.11.86 DE 3640614**

(43) Veröffentlichungstag der Anmeldung:
**08.06.88 Patentblatt 88/23**

(45) Bekanntmachung des Hinweises auf die Patenterteilung:
**29.01.92 Patentblatt 92/05**

(84) Benannte Vertragsstaaten:
**AT BE CH DE ES FR GB IT LI LU NL SE**

(56) Entgegenhaltungen:
**EP-A- 0 216 315**
**EP-A- 0 226 988**

(73) Patentinhaber: **HOECHST AKTIENGESELLSCHAFT**
**Postfach 80 03 20**
**W-6230 Frankfurt am Main 80(DE)**

(72) Erfinder: **Bach, Hanswilhelm, Dr. Dipl.-Chem.**
**Alleestrasse 56**
**W-4100 Duisburg 11(DE)**
Erfinder: **Cornils, Boy, Dr. Dipl.-Chem.**
**Kirschgartenstrasse 6**
**W-6238 Hofheim(DE)**
Erfinder: **Gick, Wilhelm, Dr. Dipl.-Chem.**
**Im Buschhuck 8**
**W-4100 Duisburg 74(DE)**
Erfinder: **Hahn, Heinz-Dieter, Dr. Dipl.-Chem.**
**Burgstrasse 21**
**W-4200 Oberhausen 11(DE)**
Erfinder: **Konkol, Werner, Dr. Dipl.-Chem.**
**Lützowstrasse 40 a**
**W-4200 Oberhausen 11(DE)**
Erfinder: **Wiebus, Ernst**
**Ferdinandstrasse 77**
**W-4200 Oberhausen 11(DE)**

**EP 0 269 964 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein verbessertes Verfahren zur Herstellung von Aldehyden durch Hydroformylierung von Olefinen unter Verwendung von in Wasser gelösten Rhodium-Komplexverbindungen als Katalysator.

Aus der DE 26 27 354 C3 ist ein Verfahren zur Herstellung von Aldehyden durch Umsetzung von aliphatischen $C_2$- bis $C_{20}$-Olefinen mit Kohlenmonoxid und Wasserstoff in flüssiger Phase in Gegenwart von Wasser sowie von Rhodium in metallischer Form oder in Form einer seiner Verbindungen, eines Arylphosphins und von Alkalimetall-, Erdalkalimetall-oder Ammoniumionen bekannt. Es ist dadurch gekennzeichnet, daß man die Umsetzung in wäßriger Lösung in Gegenwart eines wasserlöslichen, sulfonierten Aryl- oder Napthylphosphins durchführt.

Nach einem in der DE 31 35 127 Al beschriebenen Verfahren setzt man als wasserlösliche Phosphine Verbindungen ein, die in den Aryl- oder Napthylgruppen Carboxylreste enthalten.

Die Hydroformylierung von Olefinen in Gegenwart einer wäßrigen Katalysatorphase hat eine Reihe Vorteile. So erfolgt die Abtrennung des Katalysators vom Reaktionsprodukt nach Beendigung der Hydroformylierungsreaktion bei dieser Verfahrensvariante einfach durch Trennung von wäßriger und organischer Phase, d.h. ohne Destillation und damit ohne zusätzliche thermische Belastung der Reaktionsprodukte. Ferner zeichnet sich das Verfahren durch hohe Selektivität hinsichtlich der Bildung von n-Verbindungen gegenüber iso-Verbindungen aus. So enthält das bei der Hydroformylierung von Propylen anfallende Reaktionsprodukt etwa 95 Gew.-% n-Butyraldehyd und nur 5 Gew.-% des für eine Weiterverwendung weniger gefragten iso-Butyraldehyds. Schließlich wird auch eine Vergiftung des Katalysators durch hochsiedende Nebenprodukte - sie entstehen z.B. durch Aldolisierung bzw. Aldolkondensation oder Acetalbildung - weitgehend vermieden.

Die Reaktion des Olefins mit Kohlenmonoxid und Wasserstoff verläuft in der wäßrigen, der katalysatorhaltigen Phase. Nach der deutschen Patentanmeldung P 35 46 123.3 enthält die Katalysatorlösung 450° bis 800 Gew.-ppm Rhodium und 25 bis 30 Gew.-% sulfonierte oder carboxylierte Triarylphosphine, jeweils bezogen auf die wäßrige Lösung. Das Verhältnis von Rhodium zu Phosphin (in g-Atom je Mol) beträgt üblicherweise 1 : 10 bis 300, bevorzugt 1 : 50 bis 150.

Ein Maß für die Wirksamkeit des aus Rhodium und wasserlöslichem Liganden bestehenden Katalysatorsystems ist die Anzahl der Mole Aldehyd, die je Volumeinheit Katalysatorlösung und je Zeiteinheit gebildet werden. Für diese Beziehung wird im folgenden der Begriff "Produktivität" verwendet, also

$$\text{Produktivität} = \frac{\text{Mole Aldehyd}}{\text{l Katalysatorlösung} \cdot \text{h}}$$

Mit steigender Rhodiummenge in der wäßrigen Katalysatorlösung wird die Produktivität erhöht. Darüber hinaus beeinflußt die Rhodiumkonzentration aber auch die Beständigkeit der sulfonierten oder carboxylierten Triarylphospine. Mit zunehmender Rhodiumkonzentration wächst die Tendenz, daß die Kohlenstoff-Phosphor-Bindung z.B. unter Bildung substituierter Phosphinsäurederivate, Arylsulfonate oder Arylcarboxylate aufspaltet. Diese Reaktion hat zur Folge, daß die selektive Wirkung des Katalysatorsystems zurückgeht und vermehrt iso-Verbindung gebildet wird. Die Abnahme der selektiven Wirkung kann sich weiterhin darin zeigen, daß vermehrt Alkohole und höhersiedende Kondensationsprodukte gebildet werden.

Die Produktivität des Katalysatorsystems ist außer von der Rhodiumkonzentration auch von der Konzentration der sulfonierten oder carboxylierten Triarylphosphine in der Katalysatorlösung abhängig. Eine Zunahme des Phosphinanteils in der Wasserphase über 28 Gew.-% und insbesondere über 30 Gew-%, bezogen auf die wäßrige Lösung, führt zu einer Minderung der Geschwindigkeit der Hydroformylierungsreaktion und damit zu einem Rückgang der Produktivität des Katalysatorsystems.

Auch bei Einhaltung der in der deutschen Patentanmeldung P 35 46 123.3 genannten Rhodium- bzw. Phosphinkonzentrationen werden die Phosphine im Laufe der Zeit unter Spaltung der Kohlenstoff-Phosphor-Bindungen aber auch durch Oxidationsvorgänge umgewandelt. Die Phosphinkonzentration sinkt mit der bereits beschriebenen Folge, daß sich bei Verwendung lange gebrauchter Katalysatorlösungen das n-/iso-Verhältnis der Produkte im Reaktionsgemisch zugunsten der Iso-Verbindungen verändert. Daher ist es erforderlich, in gewissen Zeitabständen die Katalysatorlösung zu erneuern.

Es bestand somit die Aufgabe, einen Weg zu finden, die ursprünglichen Katalysatoreigenschaften, also insbesondere Aktivität, Produktivität entsprechend der oben wiedergegebenen Definition und selektive Wirkung über einen möglichst langen Zeitraum aufrechtzuerhalten.

Erfindungsgemäß wird diese Aufgabe gelöst durch ein Verfahren zur Herstellung von Aldehyden durch Umsetzung von aliphatischen Olefinen mit 2 bis 12 Kohlenstoffatomen mit Kohlenmonoxid und Wasserstoff bei 20 bis 150 °C und 0,1 bis 20 MPa in flüssiger Phase und in Gegenwart einer wäßrigen Lösung als Katalysator, die ursprünglich 50 bis 800, vorzugsweise 200 bis 600 Gew.-ppm Rhodium und 25 bis 30 Gew.-% komplexbildende, sulfonierte oder carboxylierte Triarylphosphine, jeweils bezogen auf die wäßrige Lösung, enthält. Es ist dadurch gekennzeichnet, daß zur Aufrechterhaltung der ursprünglichen Phosphinkonzentration in der wäßrigen Lösung des Katalysators solange frisches wasserlösliches Phosphin zugeführt wird, bis die Gesamtkomzentration an komplexbildenden Phosphinen und zur Komplexbildung nicht fähigen Folge- und Abbauprodukten der Phosphine 35 bis 45 Gew.-%, bezogen auf die wäßrige Lösung, beträgt.

Die neue Arbeitsweise stellt sicher, daß sich die Wirksamkeit des Katalysators auch nach langem Gebrauch nicht oder nur geringfügig von der eines frischen Katalysators unterscheidet. Unter dem Begriff Wirksamkeit werden vor allem die Produktivität und die selektive Wirkung des Katalysators verstanden.

Es war insbesondere nicht vorauszusehen, daß im Falle einer über einen längeren Zeitraum eingesetzten Katalysatorlösung, die Gesamtkonzentration an Phosphinen und Phosphin-Folge- und Abbauprodukten auf Werte oberhalb von 25 bis 30 Gew.-% angehoben werden kann. Wie bereits gesagt wurde, führen bei frischen Katalysatorlösungen Phosphinkonzentrationen, die den genannten Bereich übersteigen, zu einer deutlichen Abnahme der Produktivität des Katalysatorsystems.Auch in der frischen Katalsatorlösung enthaltene, bezüglich der Umsetzung jedoch inerte Salze wie $Na_2SO_4$, mindern ihre Produktivität. Enthalten also zwei frische Lösungen Rhodium und Phosphine in der gleichen Konzentration, die Phosphine dabei im Bereich von 25 bis 30 Gew.-%, und sind in einer dieser beiden Lösungen darüber hinaus noch weitere Salze gelöst, so daß die Gesamtkonzentration der gelösten Salze 25 bis 30 Gew.-% übersteigt, dann weist diese konzentriertere Lösung eine geringere Produktivität auf als die andere, weniger konzentrierte. In diesem Zusammenhang ist es überraschend, daß in Katalysatorlösungen vorhandene Folgeprodukte der wasserlöslichen Phosphine, die durch Abbau-, Umlagerungs- und andere Reaktionen entstanden sind, im Gegensatz zu z.B. $Na_2SO_4$ die Produktivität der Katalysatorlösung nicht mindern, auch wenn durch ihre Gegenwart die Gesamtkonzentration aller gelösten Stoffe den Bereich von 25 bis 30 Gew.-% überschreitet.

Die wasserlöslichen, sulfonierten oder carboxylierten Phosphinliganden folgen der allegemeinen Formel

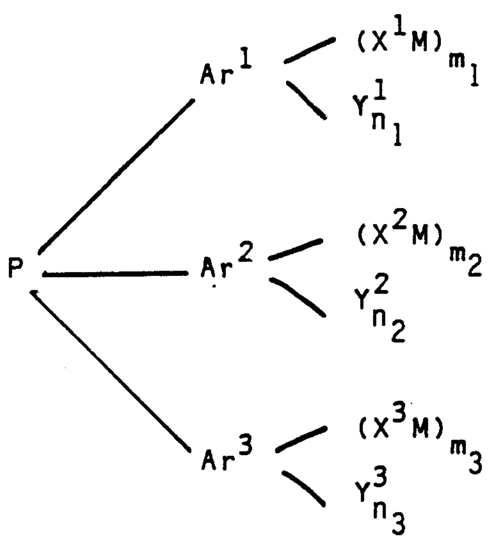

In ihr bedeuten $Ar^1$, $Ar^2$, $Ar^3$ jeweils eine Phenyl- oder Napthylgruppe, $Y^1$, $Y^2$, $Y^3$ jeweils eine geradkettige oder verzweigte Alkylgruppe mit 1 bis 4 C-Atomen, eine Alkoxygruppe, ein Halogenatom, die OH-, CN-, $NO_2$- oder $R^1R^2N$-Gruppe, in der $R^1$ und $R^2$ für jeweils eine geradkettige oder verzweigte Alkylgruppe mit 1 bis 4 C-Atomen stehen; $X^1$, $X^2$, $X^3$ ist jeweils ein Carboxylat-($COO^-$-) und/oder ein Sulfonat ($SO_3^-$-)Rest, $m_1$, $m_2$, $m_3$ sind gleiche oder verschiedene ganze Zahlen von 0 bis 3, wobei mindestens eine Zahl $m_1$, $m_2$ oder $m_3$ gleich oder größer 1 ist; $n_1$, $n_2$, $n_3$ sind gleiche oder verschiedene ganze Zahlen von 0 bis 5, M ist ein Alkalimetallion, ein Äquivalent eines Erdalkalimetall- oder Zinkions, ein Ammonium- oder quaternäres Ammoniumion der allgemeinen Formel $N(R^3R^4R^5R^6)^+$, in der $R^3$, $R^4$, $R^5$, $R^6$ jeweils für eine geradkettige oder verzweigte Alkylgruppe mit 1 bis 18 C-Atomen oder eine Aralkylgruppe mit 7 bis 14 C-Atomen steht. Bewährt haben sich insbesondere quaternäre Ammoniumgruppen, in denen

drei der Reste $R^3$, $R^4$, $R^5$, $R^6$ jeweils 1 bis 4 Kohlenstoffatome enthalten und der vierte Rest eine Aralkylgruppe mit 7 bis 14 C-Atomen ist.

Bevorzugt werden wasserlösliche Triarylphosphine der vorstehend beschriebenen allgemeinen Formel, in der $Ar^1$, $Ar^2$, $Ar^3$, jeweils einen Phenylrest, $m_1$, $m_2$ und $m_3$ 0 oder 1 bedeuten und die Summe $m_1 + m_2 + m_3$ 2 oder 3 ist und $n_1$, $n_2$ und $n_3$ 0 sind. Beispiele für Verbindungen der oben wiedergegebenen allgemeinen Formel sind Triphenylphosphin-tri-Na-trisulfonat, Triphenylphosphin-tri-(tetraalkylammonium)-trisulfonat, Triphenylphosphin-tri-Na-tricarboxylat, Tri-phenylphosphin-di-Na-sulfonat.

Die sulfonierten oder carboxylierten Arylphosphine können als einheitliche Verbindungen eingesetzt werden. Es können aber auch Gemische aus Phosphinen verwendet werden, die unterschiedlich viele Sulfonatgruppen oder Carboxylatgruppen enthalten, also z.B. Gemische aus Salzen der Triarylphosphintrisulfonsäuren und Triarylphosphindisulfonsäuren. Darüber hinaus müssen die Sulfonate oder Carboxylate nicht dasselbe Kation enthalten. Auch Gemische aus Salzen, die sich von unterschiedlichen Metallen ableiten und/oder Ammonium- und/oder quaternäre Alkylammoniumionen enthalten, sind geeignet.

Das aus Rhodium-Komplexverbindung und wasserlöslichen Phosphinen im Überschuß bestehende Katalysatorsystem wird als wäßrige Lösung eingesetzt, die 50 bis 800 Gew.-ppm Rhodium und 25 bis 30 Gew.-% wasserlösliches Phosphin, jeweils bezogen auf die Lösung, enthält. Besonders bewährt hat es sich mit Lösungen zu arbeiten, die 200 bis 600 Gew.-ppm. Rhodium und 26 bis 28 Gew.-% wasserlösliches Phosphin enthalten.

Unter der ursprünglichen Katalysatorlösung wird eine neu hergestellte Lösung verstanden, die noch keine Phosphin-Folge- und Abbauprodukte enthält oder eine gebrauchte Lösung, die Phosphin-Folge- und Abbauprodukte in so geringer Konzentration aufweist, daß ihre ursprüngliche Wirksamkeit, charakterisiert insbesondere durch die Produktivität und das Verhältnis von n- zu iso-Verbindung im Reaktionsprodukt, nicht verändert ist.

Läßt die Wirksamkeit der Katalysatorlösung nach, so führt man ihr bis zum Erreichen des Anfangszustandes wasserlösliches Phosphin zu. Selbstverständlich kann man auf diese Weise auch jedes zwischen Anfangs- und aktuellem Zustand gelegene Niveau einstellen. Welche Bezugsgröße zur Beurteilung der Wirksamkeit des Katalysatorsystems gewählt wird, ist gleichgültig. Man kann z.B. auf die selektive Bildung von n- und iso-Verbindung im Reaktionsprodukt beziehen oder auf den Anteil von Alkoholen oder höhersiedenden Kondensationsprodukten. Es ist auch möglich, mehrere Merkmale z.B. zwei oder drei zur Beurteilung des Katalysatorzustandes heranzuziehen.

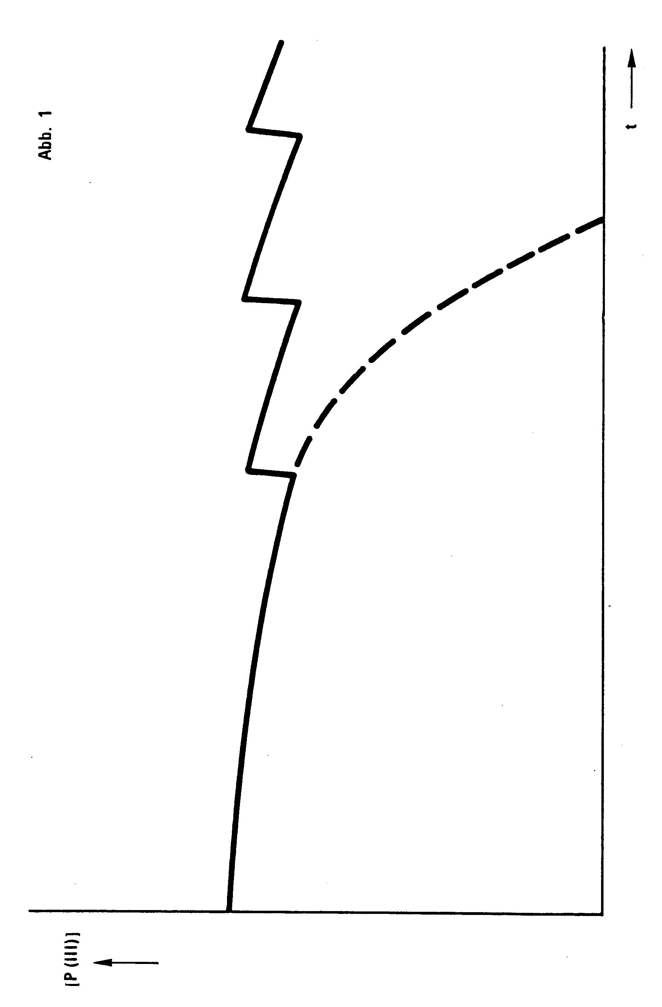

Das wasserlösliche Phosphin kann der Katalysatorlösung als Feststoff oder in Wasser gelöst zugesetzt werden. Die Konzentration des Phosphins in der wäßrigen Lösung ist dabei in weiten Bereichen frei wählbar. Bewährt hat es sich, Lösungen einzusetzen, die 25 bis 35 Gew.-% Phosphin enthalten. Das dem Katalysatorsystem zugesetzte Phosphin braucht nicht mit dem übereinzustimmen, das bereits in der Katalysatorlösung enthalten ist. Sie können sich sowohl durch Kation als auch durch Anion oder durch Kation und Anion unterscheiden. So kann man einer Lösung, die ursprünglich nur Triphenylphosphin-tri-Na-trisulfonat enthielt, das entsprechende Kaliumsalz oder ein Tetraalkylammoniumsalz oder ein Salz der Triphenylphosphin-disulfonsäure zusetzen. Die Zugabe kann kontinuierlich oder diskontinuierlich erfolgen. Ein für die diskontinuierliche Arbeitsweise charakteristischer Verlauf der Phosphin-Konzentration, in Abhängigkeit von der Zeit, ist in der beigefügten Abbildung dargestellt.

Die Zugabe von Phosphin zur Katalysatorlösung kann fortgesetzt werden, bis die Gesamtkonzentration an Phosphin und Phosphin-Folge- bzw. Abbauprodukten 35 bis 45 Gew.-%, insbesondere 40 bis 45 Gew.-%, bezogen auf die wäßrige Lösung. beträgt. Ein weiterer Phosphinzusatz vermag die Wirksamkeit der Katalysatorlösung nicht mehr auf den ursprünglichen Zustand zurückzuführen.

Nach Erreichen der maximalen Gesamtkonzentration kann die Katalysatorlösung bei nachlassender Selektivität insgesamt oder durch kontinuierliche Entnahme von Teilen der Lösung aufgearbeitet werden.

Ein geeignetes Aufarbeitungsverfahren ist z.B. die Extraktion der zuvor angesäuerten Lösung mit der Lösung eines Amins in einem organischen Lösungsmittel und anschließende Behandlung der organischen Phase mit der wäßrigen Lösung einer anorganischen Base. Nach einer anderen Arbeitsweise trennt man Rhodium-Komplexverbindung und Phosphine einschließlich ihrer Umwandlungsprodukte nach einem Membrantrennverfahren. Die Rhodium-Komplexverbindung kann unmittelbar wieder als Katalysatorbestandteil eingesetzt werden, während das Phosphin durch Extraktion mit Amin wiedergewonnen wird.

Nach dem erfindungsgemäßen Verfahren lassen sich Olefine mit 2 bis 12 Kohlenstoffatomen hydroformylieren. Diese Olefine können linear oder verzweigt sein und eine endständige oder innenständige Doppelbindung enthalten. Ebenso können Cycloolefine mit 6 bis 12 Kohlenstoffatomen umgesetzt werden. Beispiele für die vorstehend beschriebenen Olefine sind: Ethylen, Propylen, i-Buten, 2-Buten, 1-Penten, 2-Methyl-1-buten, 4,4-Dimethyl-1-nonen, 1-Dodecen, Cyclohexen, Dicyclopentadien. Bevorzugt werden lineare Olefine mit 2 bis 8 Kohlenstoffatomen wie Ethylen, Propylen, 1-Buten, 1-Penten, 1-Hexen, 1-Hepten und 1-Octen sowie als Cycloolefin Dicyclopentadien eingesetzt.

Der Gesamtdruck von Wasserstoff und Kohlenmonoxid beträgt 0,1 bis 20 MPa, vorzugsweise 1 bis 10 MPa. Die Zusammensetzung des Synthesegases, d.h. das Verhältnis von Kohlenmonoxid zu Wasserstoff kann in weiten Grenzen variiert werden. Im allgemeinen setzt man Synthesegas ein, in dem das Volumverhältnis von Kohlenmonoxid zu Wasserstoff 1 : 1 beträgt oder von diesem Wert nur wenig abweicht. Die Umsetzung erfolgt bei Temperaturen von 20 bis 150 °C, sie kann sowohl kontinuierlich als auch absatzweise durchgeführt werden.

In den nachfolgenden Beispielen wird eine Ausführungsform der Erfindung näher erläutert.

Beispiel 1 (Vergleichsbeispiel)

In eine wäßrige Katalysatorlösung, die 27 Gew.-% eines Gemisches der Natriumsalze der Triphenylphosphintrisulfonsäure und der Triphenylphosphindisulfonsäure sowie 500 Gew.-ppm Rhodium, jeweils bezogen auf die Lösung, enthält, werden unter Rühren bei einer Temperatur von 122 °C und einem Druck von 5 MPa Propylen, Kohlenmonoxid und Wasserstoff im Volumverhältnis 1 : 1 : 1 geleitet. Je Liter Katalysatorlösung und Stunde erhält man 1,95 Mole eines Gemisches aus 95 % n- und 5 % iso-Butyraldehyd. Im Laufe der Zeit wird der Phosphinligand allmählich abgebaut und die Selektivität der Reaktion wird schlechter.

Beispiel 2

Beispiel 1 wird wiederholt. Sobald geringe Selektivitätseinbußen beobachtet werden, d.h. bei einem Phosphin/Rh-Verhältnis von 90 : 1 (Mol : g-Atom), wird frischer Ligand in einer solchen Menge zugegeben, daß das ursprüngliche Phosphin/Rh-Verhältnis wieder erreicht wird.

Dieser Vorgang kann so lange ohne Produktivitätsrückgang wiederholt werden, bis die Gesamtkonzentration von Phosphin und Phosphin-Folge- und Abbauprodukten 45 Gew.-%, bezogen auf die Lösung, beträgt. Während der gesamten Zeit wird ein Aldehydgemisch mit konstanter Zusammensetzung (95 % n-, 5 % iso-Verbindung) erhalten.

**Patentansprüche**

1. Verfahren zur Herstellung von Aldehyden durch Umsetzung von aliphatischen Olefinen mit 2 bis 12 Kohlenstoffatomen mit Kohlenmonoxid und Wasserstoff bei 20 bis 150 °C und 0,1 bis 20 MPa in flüssiger Phase und in Gegenwart einer wäßrigen Lösung als Katalysator, die ursprünglich 50 bis 800, vorzugsweise 200 bis 600 Gew.-ppm Rhodium und 25 bis 30 Gew.-% komplexbildende, sulfonierte oder carboxylierte Triarylphosphine, jeweils bezogen auf die wäßrige Lösung, enthält, dadurch gekennzeichnet, daß zur Aufrechterhaltung der ursprünglichen Phosphinkonzentration in der wäßrigen Losung des Katalysators solange frisches wässerlösliches Phosphin zugeführt wird, bis die Gesamtkonzentration an komplexbildenden Phosphinen und zur Komplexbildung nicht fähigen Folge- und Abbauprodukten der Phosphine 35 bis 45 Gew.-%, bezogen auf die wäßrige Lösung, beträgt.

2. Verfahren nach Anspruch 1, dadurch gekennzeichnet, daß das wasserlösliche Phosphin der Katalysatorlösung als Feststoff zugesetzt wird.

3. Verfahren nach Anspruch 1, dadurch gekennzeichnet, daß das wasserlösliche Phosphin der Katalysatorlösung als wäßrige Lösung zugesetzt wird.

4. Verfahren nach Anspruch 3, dadurch gekennzeichnet, daß die wäßrige Lösung 26 bis 28 Gew.-% Phosphin enthält.

5. Verfahren nach einem oder mehreren der Ansprüche 1 bis 4, dadurch gekennzeichnet, daß die Zugabe der wasserlöslichen Phosphins kontinuierlich erfolgt.

6. Verfahren nach einem oder mehreren der Ansprüche 1 bis 4, dadurch gekennzeichnet, daß das wasserlösliche Phosphin der Katalysatorlösung diskontinuierlich zugesetzt wird.

7. Verfahren nach einem oder mehreren der Ansprüche 1 bis 6, dadurch gekennzeichnet, daß die Gesamtkonzentration an komplexbildenden Phosphinen und zur Komplexbildung nicht fähigen Folge-

und Abbauprodukten der Phosphine schließlich 40 bis 45 Gew.-%, bezogen auf die wäßrige Lösung, beträgt.

## Claims

1. A process for the preparation of aldehydes by the reaction of aliphatic olefins having 2 to 12 carbon atoms with carbon monoxide and hydrogen at temperatures of 20 to 150 °C and pressures of 0.1 to 20 MPa in the liquid phase and in the presence of an aqueous solution as a catalyst containing originally 50 to 800, preferably 200 to 600 wt. ppm rhodium and 25 to 30 wt. % complex-forming, sulfonated or carboxylated triarylphosphines, in each case related to the aqueous solution, characterised in that, in order to maintain the original phosphine concentration in the aqueous solution of the catalyst, fresh water-soluble phosphine is added until the total concentration of complex-forming phoshines and secondary and degradation products of the phosphines not capable of forming complexes amounts to about 35 to 45 wt. %, related to the aqueous solution.

2. A process according to claim 1, characterised in that the water-soluble phosphine of the catalyst solution is added as a solid.

3. A process according to claim 1, characterised in that the water-soluble phosphine of the catalyst solution is added as an aqueous solution.

4. A process according to claim 3, characterised in that the aqueous solution contains 26 to 28 wt. % phosphine.

5. A process according to one or several of the claims 1 to 4, characterised in that the water-soluble phosphine is added continuously.

6. A process according to one or several of the claims 1 to 4, characterised in that the water-soluble phosphine is added discontinuously to the catalyst solution.

7. A process according to one or several of the claims 1 to 6, characterised in that the total concentration of complex-forming phosphines and secondary and degradation products of the phosphines not capable of forming complexes finally amounts to 40 to 45 wt. % related to the aqueous solution.

## Revendications

1. Procédé pour la fabrication d'aldéhydes par réaction d'oléfines aliphatiques en $C_2$-$C_{12}$ avec le monoxyde de carbone et l'hydrogène à 20-150 °C et sous 0,1-20 MPa en phase liquide et en présence d'une solution aqueuse comme catalyseur qui contient initialement 50 à 800 ppm, de préférence 200 à 600 ppm en poids de rhodium et 25 à 30 % en poids de triarylphosphines sulfonées ou carboxylées formant des complexes, rapportés chaque fois à la solution aqueuse, caractérisé en ce que, pour le maintien de la concentration initiale en phosphine, on envoie dans la solution aqueuse du catalyseur de la phosphine soluble dans l'eau fraîche jusqu'à ce que la concentration totale en phosphines formant des complexes et dérivés et produits de dégradation des phosphines ne formant pas de complexes, soit de 35 à 45 % en poids, par rapport à la solution aqueuse.

2. Procédé selon la revendication 1, caractérisé en ce que la phosphine soluble dans l'eau est ajoutée sous forme solide à la solution de catalyseur.

3. Procédé selon la revendication 1, caractérisé en ce que la phosphine soluble dans l'eau est ajoutée sous forme de solution aqueuse à la solution de catalyseur.

4. Procédé selon la revendication 3, caractérisé en ce que la solution aqueuse contient 26 à 28 % en poids de phosphine.

5. Procédé selon une ou plusieurs des revendications 1 à 4, caractérisé en ce que l'addition de la phosphine soluble dans l'eau s'effectue en continu.

6. Procédé selon une ou plusieurs des revendications 1 à 4, caractérisé en ce que la phosphine soluble dans l'eau est ajoutée en discontinu à la solution de catalyseur.

7. Procédé selon une ou plusieurs des revendications 1 à 6, caractérisé en ce que la concentration totale finale en phosphines formant des complexes et dérivés et produits de dégradation des phosphines ne formant pas de complexes est de 40 à 45 % en poids par rapport à la solution aqueuse.

Abb. 1